# EUROPEAN PATENT SPECIFICATION

(11) **EP 3 192 490 B1**
(45) Date of publication and mention of the grant of the patent: **01.05.2019**
(21) Application number: 17151109.0
(22) Date of filing: 12.01.2017
(51) Int. Cl.: A61K 8/25, A61Q 19/10, A61K 8/92, A61K 8/20

(54) **BODY SCRUB COMPOSITIONS**
KÖRPERPEELINGZUSAMMENSETZUNGEN
COMPOSITIONS DE LOTION EXFOLIANTE

(30) Priority: 13.01.2016 US 201614994200
(43) Date of publication of application: 19.07.2017
(73) Proprietor: Henkel IP & Holding GmbH, 40589 Düsseldorf (DE)
(72) Inventor: KAYIRAN, Isil, WA 98119, Seattle, (US); ALVAREZ, Terannie Vazquez, AZ 85295, Gilbert, (US); CONWAY, Mary J, AZ 85022, Phoenix, (US); LUCIOW, Chris, Valley, AZ 85253, Paradise (US)
(74) Representative: Augustin-Castro, Barbara

(56) References cited:
- WO-A1-2010/123559
- WO-A2-00/04867
- US-A1- 2004 028 630
- US-B1- 7 101 578

## Description

### TECHNICAL FIELD

The technical field relates generally to body scrub compositions and methods for forming body scrub compositions, and more particularly relates to body scrub compositions including natural oil blends.

### BACKGROUND

Human skin naturally creates a protective cream-like layer made of sebum, lecithin, cholesterol and water. Bathing daily with chlorinated municipal water and harsh soaps strips the skin of its natural protective layer. To overcome this problem and hydrate the skin, people use oils or creams and lotions composed of water, emulsifiers and emollients, i.e., oils, fats, or lipids. However, such treatments do not provide deep moisturizing of the skin, nor do they replace the protective layer which has been stripped. Exfoliation, the practice of rubbing coarse grains over the skin for cleansing is older than soap, and various compositions have been developed for that purpose. Exfoliation is the removal of dead skin cells from the outer dermal layers. Exfoliation can be achieved by rubbing the skin with a washcloth, loofah, pumice stone or other tool, or with loose grains such as ground nut shells or pits, ground pumice, sugar, salt or oatmeal, or with chemicals such as alpha hydroxy acids or fruit acids that loosen and break down the outer dermal layer.

There are different types of commercial body scrub compositions including exfoliants. A first type of body scrub composition contains a high level of oils, such as more than 50 wt.% oils. The high concentration of oils provides soft and smooth skin after use. However, these formulations typically suffer from layer separation. Specifically, they often separate into a top layer containing oils and a bottom layer rich in a base, such as sugar or salt, and exfoliating particles. Separation of the two layers results in inconvenience during usage. Typically, such body scrub needs to be physically mixed to obtain a representative sample for use. In addition, this type of composition often leaves an oily residue on the surface of the tub or shower.

A second type of body scrub composition employs a gelling agent such as silica to achieve a homogenous appearance. This type of composition typically contains low amounts of oil, such as less than about 15 wt.%, and/or high levels of gelling agent, such as greater than 20 wt.%. Such a formulation strategy results in low levels of moisturization and decreased softness and smoothness on the skin.

US7101578 discloses salt sorbet facial and body scrub comprising sea salt, oils and 7.4- 9.05 wt % silica. WO0004867 concerns skin scrub compositions comprising sea salt and an oil blend but no silica.

Accordingly, it is desirable to provide body scrub compositions that exhibit a homogenous appearance without separation. Further, it is desirable to provide body scrub compositions that exhibit good oil deposition properties. Also, it is desirable to provide body scrub compositions that are low cost. Furthermore, other desirable features and characteristics will become apparent from the subsequent detailed description and the appended claims, taken in conjunction with the accompanying drawings and the foregoing technical field and background.

### BRIEF SUMMARY

Body scrub compositions and methods for forming body scrub compositions are provided herein. In one embodiment, a body scrub composition includes safflower oil, argan oil, and marula oil and includes silica as a suspension agent. The body scrub of the invention is defined as set out in claim 1.

In accordance with another exemplary embodiment, a body scrub composition includes a suspension agent. The body scrub composition also includes an exfoliant. Further, the body scrub composition includes a combination of natural oils including safflower oil, argan oil, and marula oil.

In accordance with another exemplary embodiment, a method for forming a body scrub composition is provided. The method includes blending safflower oil, argan oil, and marula oil to form an oil blend. Also, the method includes adding a suspension agent to the oil blend.

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

### DETAILED DESCRIPTION

The following detailed description is merely exemplary in nature and is not intended to limit the exfoliating body scrub compositions described herein. Furthermore, there is no intention to be bound by any expressed or implied theory presented in the preceding technical field, background or brief summary, or in the following detailed description.

The various embodiments contemplated herein relate to body cleansing compositions, and particularly to exfoliating body scrub compositions. Exemplary exfoliating body scrub compositions include a unique combination of oils, such as safflower oil, marula oil, and argan oil.

Body scrub compositions herein achieve homogenous appearance and good oil deposition properties at low cost. Such compositions have an oil content high enough to provide oil deposition properties and low enough to keep a homogenous appearance. Further, such compositions have a silica content high enough to keep all ingredients in one homogenous phase and low enough to prevent dry skin caused by silica particles. Also, such compositions have an exfoliant content high enough to assure exfoliation feeling and low enough to keep a homogenous appearance. Additionally, use of lower cost materials such as safflower oil as the main oil, regular table salt as the base, and larger sea salt particles as exfoliant provide for low cost formulation.

In an exemplary embodiment, the composition appears homogenous upon preparation. The composition remains mostly homogenous, i.e., less than about 10 wt.% of fragrance oil separates from the remaining composition, during tested durations such as during a time period of 12 weeks at a temperature of 4 °C, during a time period of 12 weeks at a temperature of 25 °C, during a time period of 12 weeks at a temperature of 40 °C, during a time period of 2 weeks at a temperature of 50 °C, or during a time period of 1 week at a temperature of 60 °C.

In an exemplary embodiment, the composition deposits oil on skin. In an exemplary embodiment, the composition is moisturizing. In an exemplary embodiment, the composition provides softer and smoother skin after first use. In an exemplary embodiment, the composition is a low cost due to the use of safflower oil, table salt, sea salt exfoliant, and a low amount of silica, such as less than about 5.0 wt.%.

Exemplary exfoliating body scrub compositions have a homogenous appearance. An exemplary composition has an oil content low enough to keep a homogenous appearance after formation. For example, the composition may have an oil content of less than about 50 wt.%, for example less than about 40 wt.%, such as less than 30 wt.%, based on the total weight of the composition. In an exemplary embodiment, the composition has an oil content of less than about 27 wt.%, such as about 26.2 wt.%, based on the total weight of the composition. In another exemplary embodiment, the composition has an oil content of less than about 25 wt.%, for example less than about 20 wt.%, such as less than 15 wt.%, based on the total weight of the composition. In an exemplary embodiment, the composition has an oil content of less than about 10 wt.%, for example, less than about 7.5 wt.%, such as about 5.1 wt.%, based on the total weight of the composition.

Further, exemplary exfoliating body scrub compositions have good oil deposition properties. An exemplary composition has an oil content high enough to provide for good oil deposition during use. For example, the composition may have an oil content of greater than about 1 wt.%, for example greater than about 2.5 wt.%, such as greater than about 4 wt.%, based on the total weight of the composition. In an exemplary embodiment, the composition has an oil content of greater than about 5 wt.%, such as about 5.1 wt.%, based on the total weight of the composition. In another exemplary embodiment, the composition may have an oil content of greater than about 10 wt.%, for example greater than about 15 wt.%, such as greater than about 20 wt.%, based on the total weight of the composition. In an exemplary embodiment, the composition has an oil content of greater than about 25 wt.%, such as about 26.2 wt.%, based on the total weight of the composition.

The exfoliating body scrub compositions include a suspension agent content high enough to keep all ingredients in one homogenous phase. For example, an exemplary composition has a suspension agent content of greater than 0.5 wt.%, for example greater than about 0.8 wt.%, such as greater than about 1 wt.%, based on the total weight of the composition. In an exemplary embodiment, the composition has a suspension agent content of greater than about 1.5 wt.%, such as about 2 wt.%, based on the total weight of the composition.

Further, exemplary exfoliating body scrub compositions may include a suspension agent content low enough to prevent dry skin caused by suspension agent particles. For example, the composition may have a suspension agent content of less than 2 wt.%, for example less than about 1.5 wt.%, such as less than 1 wt.%, based on the total weight of the composition. In an exemplary embodiment, the composition has a suspension agent content of less than about 0.8 wt.%, for example, such as about 0.5 wt.%, based on the total weight of the composition.

The exfoliating body scrub compositions may have an exfoliant content high enough to assure exfoliation feeling. For example, the composition may have an exfoliant content of greater than about 1 wt.%, for example greater than about 2.5 wt.%, such as greater than about 4 wt.%, based on the total weight of the composition. In an exemplary embodiment, the composition has an exfoliant content of greater than about 5 wt.%, for example greater than about 8 wt.%, such as greater than about 10 wt.%, based on the total weight of the composition. In an exemplary embodiment, the composition has an exfoliant content of greater than about 12 wt.%, such as about 15 wt.%, based on the total weight of the composition.

The exfoliating body scrub compositions may have an exfoliant content low enough to keep a homogenous appearance. For example, the composition may have an exfoliant content of less than about 25 wt.%, for example less than about 20 wt.%, such as less than 15 wt.%, based on the total weight of the composition. In an exemplary embodiment, the composition has an exfoliant content of less than about 12 wt.%, for example less than about 10 wt.%, such as less than 8 wt.%, based on the total weight of the composition. In an exemplary embodiment, the composition has an exfoliant content of less than about 6 wt.%, for example, such as about 5 wt.%, based on the total weight of the composition.

Also, exemplary exfoliating body scrub compositions are provided at low cost herein through the use of lower cost materials such as safflower oil as the main oil source, regular table salt as the base, and larger sea salt particles as exfoliant. As used herein, a main oil source is the greatest constituent oil in a composition.

The exfoliating body scrub composition includes safflower oil as the main oil source. Safflower oil is extracted from seeds of the safflower tree (*Carthamus tinctorius*, asteraceae family). An exemplary composition includes from about 5.0 to about 26 wt.% safflower oil, based on the total weight of the composition.

The exfoliating body scrub composition includes marula oil. Marula oil is extracted from seeds of the marula tree (*Sclerocarya birrea*, anacardiaceae family). An exemplary composition includes from about 0.01 to about 1 wt.% marula oil, based on the total weight of the composition. For example, the composition may include from about 0.02 to about 0.5 wt.% marula oil, based on the total weight of the composition. In an exemplary embodiment, the composition includes from about 0.03 to about 0.2 wt.% marula oil, such as from about 0.05 to about 0.1 wt.% marula oil, based on the total weight of the composition.

The exfoliating body scrub composition includes argan oil. Argan oil is extracted from seeds of the argan tree (*Argania spinosa*, sapotaceae family). An exemplary composition includes from 0.05 to about 0.1 wt.% argan oil, based on the total weight of the composition.

The oil content of an exemplary composition consists essentially of the natural oils: safflower oil, marula oil, and argan oil. This combination of natural oils is believed to uniquely provide for sufficient oil deposition during use while avoiding separation and exhibiting a homogenous appearance after formation.

In an exemplary embodiment, the body scrub composition has a safflower oil : argan oil ratio of from about 50:1 to about 520:1. For example, the body scrub composition may have a safflower oil : argan oil ratio of at least about 50:1 or at least about 100:1, such as at least about 200:1, for example at least about 300:1. Exemplary body scrub compositions may have a safflower oil: argan oil ratio of no more than about 500:1, such as no more than 400:1, for example no more than about 300, no more than about 200:1, or no more than about 100:1.

In an exemplary embodiment, the body scrub composition has a safflower oil : marula oil ratio of from about 50:1 to about 520:1. For example, the body scrub composition may have a safflower oil : marula oil ratio of at least about 50:1 or at least about 100:1, such as at least about 200:1, for example at least about 300:1. Exemplary body scrub compositions may have a safflower oil : marula oil ratio of no more than about 500:1, such as no more than 400:1, for example no more than about 300, no more than about 200:1, or no more than about 100:1.

An exemplary body scrub composition has an argan oil : marula oil ratio of from about 0.5:1 to about 2:1. For example, an exemplary body scrub composition has an argan oil : marula oil ratio of at least about 0.5:1, such as at least about 0.8:1, such as at least about 1:1 or at least about 1.5:1. An exemplary body scrub composition has an argan oil : marula oil ratio of no more than about 2:1, such as no more than about 1.5:1, for example no more than about 1:1 or no more than about 0.8:1.

An exemplary body scrub composition has a marula oil : argan oil ratio of from about 0.5:1 to about 2:1. For example, an exemplary body scrub composition has a marula oil : argan oil ratio of at least about 0.5:1, such as at least about 0.8:1, such as at least about 1:1 or at least about 1.5:1. An exemplary body scrub composition has a marula oil : argan oil ratio of no more than about 2:1, such as no more than about 1.5:1, for example no more than about 1:1 or no more than about 0.8:1.

Exemplary exfoliating body scrub compositions include silica as a suspension agent. Other suitable suspension agents may be employed in addition to silica. An exemplary composition may have a silica content of greater than 0.5 wt.%, for example greater than about 0.8 wt.%, such as greater than about 1 wt.%, based on the total weight of the composition. In an exemplary embodiment, the composition has a silica content of less than about 2 wt.%, for example less than about 1.5 wt.%, such as less than 1 wt.%, based on the total weight of the composition. In an exemplary embodiment, the composition has a silica content of less than about 0.8 wt.%, for example, such as about 0.5 wt.%, based on the total weight of the composition.

The exfoliating body scrub compositions include an exfoliant. A suitable exfoliant for use in the composition is sea salt. Other suitable exfoliants may be employed instead of or in addition to sea salt. Exemplary crystalline sea salt grains are consistent in shape and size to effectively exfoliate without scratching the skin. The edges are sharp enough to exfoliate the outer dermal layer, without being jagged enough to break the skin's deeper layers. The salt is also antibacterial so it "cleanses" the skin. Salt crystals may lie upon each other askew enabling the oils to occupy the space in between. An exemplary sea salt is composed of about 99.95% minimum sodium chloride, 0.04% maximum sodium sulfate, 60 ppm maximum calcium and magnesium, 0.5 ppm maximum iron, and 0.4 ppm maximum copper. Exemplary salt crystals may have an average diameter or dimension of from about 0.005 to about 0.02 inches.

The composition may have an exfoliant content of greater than 5 wt.%, for example greater than about 7.5 wt.%, such as greater than about 10 wt.%, based on the total weight of the composition. In an exemplary embodiment, the composition has an exfoliant content of greater than about 12 wt.%, such as about 15 wt.%, based on the total weight of the composition. In an exemplary embodiment, the composition has an exfoliant content of less than about 15 wt.%, for example less than about 10 wt.%, such as less than 8 wt.% or 5 wt.%, based on the total weight of the composition.

The exfoliating body scrub composition includes a base that is sodium chloride. Other suitable bases may be employed in addition to table salt. The salt may be provided to adjust the viscosity of the composition. In an exemplary embodiment, the composition may have a salt content of greater than about 40 wt.%, based on the total weight of the composition. In an exemplary embodiment, the composition has a salt content of greater than about 45 wt.%, for example greater than about 50 wt.%, such as greater than about 55 wt.% or 56 wt.%, based on the total weight of the composition. In an exemplary embodiment, the composition has a salt content of less than 56 wt.%, for example less than about 50 wt.%, such as less than 45 wt.% or 40 wt.%, based on the total weight of the composition.

The exfoliating body scrub may further include an additional moisturizer. The exfoliating body scrub may further include a moisturizer. For example, a suitable moisturizer may be coconut oil, jojoba oil, mango butter, shea butter, cocoa butter, or any other specialty oils or butters or combinations thereof known for moisturizing and softening the skin. An exemplary composition may have a moisturizer content of greater than about 0.25 wt.%, for example greater than about 0.5 wt.%, such as greater than about 0.75 wt.%, based on the total weight of the composition. In an exemplary embodiment, the composition has a moisturizer content of greater than about 1 wt.%, for example greater than about 1.25 wt.%, such as greater than about 1.5 wt.% or greater than about 1.75 wt.%, for example about 2 wt.%, based on the total weight of the composition. Further, the composition may have moisturizer content of less than about 5 wt.%, for example less than about 4 wt.%, such as less than 3 wt.%, based on the total weight of the composition. In an exemplary embodiment, the composition has a moisturizer content of less than about 2 wt.%, for example less than about 1.5 wt.%, such as about 1 wt.%, based on the total weight of the composition.

The exfoliating body scrub composition may further include a preservative. For example, the composition may include a preservative in the form of vitamin E. Other suitable preservatives may be employed instead of or in addition to vitamin E. An exemplary composition may have a preservative content of greater than about 0.02 wt.%, for example greater than about 0.05 wt.%, such as greater than about 0.08 wt.%, based on the total weight of the composition. In an exemplary embodiment, the composition has a preservative content of greater than about 0.1 wt.%, for example greater than about 0.5 wt.%, such as greater than about 1 wt.% or greater than about 1.5 wt.%, for example about 2 wt.%, based on the total weight of the composition. Further, the composition may have preservative content of less than about 5 wt.%, for example less than about 4 wt.%, such as less than 3 wt.%, based on the total weight of the composition. In an exemplary embodiment, the composition has a preservative content of less than about 2 wt.%, for example less than about 1 wt.%, such as about 0.5 wt.%, based on the total weight of the composition.

An exemplary method for forming an exfoliating body scrub compositions includes forming a natural oil blend by blending the safflower oil, argan oil, and marula oil. Then the suspension agent may be added to the oil blend and mixed. Further, the exfoliant may be added and stirred into the oil blend. Other components such as moisturizer, base (salt), and preservative may be added and mixed to the oil blend before or after the addition of the exfoliant.

### Example 1

The following is an example of a body scrub composition in accordance with an exemplary embodiment. The example is provided for illustration purposes only and is not meant to limit the various embodiments of the body scrub composition in any way. All materials are set forth in weight percent, based on the total weight of the body scrub composition.

### Example 1 - Body Scrub Composition

| Ingredient | Wt% |
|---|---|
| Safflower Oil | 5.0-26.0 |
| Marula Oil | 0.05-0.1 |
| Argan Oil | 0.05-0.1 |
| Moisturizer | 1.0-2.0 |
| Regular Table Salt | 40.0-56.0 |
| Silica (Suspension agent) | 0.5-2.0 |
| Sea Salt (Exfoliant) | 5.0-15.0 |
| Vitamin E (Preservative) | 0.1-2.0 |
| Total | 100.0 |

In Example 1, the body scrub composition has a viscosity of from about 100,000 cps to about 200,000 cps, as measured by a TE spindle with helipath, at 6 RPM.

Testing has shown that after a week of daily application of the composition of Example 1 to the skin, it is possible to observe: softened and rehydrated skin, a reduction of wrinkles, and a reduction of age spots.

Accordingly, body scrub compositions have been disclosed. The body scrub compositions include a blend of natural oils including safflower oil, argan oil, and marula oil. The unique blend of oils are provided at concentrations high enough to provide proper oil deposition properties during application and low enough to maintain a homogenous appearance. Further, the body scrub compositions have a silica content high enough to keep all ingredients in one homogenous phase and low enough to prevent dry skin caused by silica particles. Also, such compositions have an exfoliant content high enough to assure exfoliation feeling and low enough to keep a homogenous appearance.

While at least one exemplary embodiment has been presented in the foregoing detailed description, it should be appreciated that a vast number of variations exist. It should also be appreciated that the exemplary embodiment or embodiments described herein are not intended to limit the scope, applicability, or configuration of the claimed subject matter in any way. Rather, the foregoing detailed description will provide those skilled in the art with a convenient road map for implementing the described embodiment or embodiments.

## Claims

1. A body scrub composition comprising:
sodium chloride as a base,
from 5.0 to 26.0 wt.% safflower oil, based on the total weight of the body scrub composition;
from 0.05 to 0.1 wt.% marula oil, based on the total weight of the body scrub composition;
from 0.05 to 0.1 wt.% argan oil, based on the total weight of the body scrub composition;
from 5.0 to 15.0 wt.% sea salt, based on the total weight of the body scrub composition; and
from 40.0 to 56.0 wt.% sodium chloride, based on the total weight of the body scrub composition.
from 0.5 to 2.0 wt.% silica as suspension agent, based on the total weight of the body scrub composition.

2. The body scrub composition of claim 1, wherein the sea salt as an exfoliant is present in the form of salt crystals with an average diameter of from 0.013 cm (0.005 inches) to 0.051 cm (0.02 inches).

3. The body scrub composition of any of claim 1 to 2, further comprising:
from 1.0 to 2.0 wt.% moisturizer, based on the total weight of the body scrub composition; and
from 0.1 to 2.0 wt.% preservative, based on the total weight of the body scrub composition.

4. The body scrub composition of claim 3, comprising
from 0.1 to 2.0 wt.% Vitamin E as preservative, based on the total weight of the body scrub composition.

5. A body scrub composition of any of claims 1 to 4, comprising a moisturizer selected from coconut oil, jojoba oil, mango butter, shea butter and cocoa butter.

## Patentansprüche

1. Körperpeeling-Zusammensetzung, umfassend:
Natriumchlorid als eine Base, von 5,0 bis 26,0 Gew.-% Safloröl, bezogen auf das Gesamtgewicht der Körperpeeling-Zusammensetzung;
von 0,05 bis 0,1 Gew.-% Marulaöl, bezogen auf das Gesamtgewicht der Körperpeeling-Zusammensetzung;
von 0,05 bis 0,1 Gew.-% Arganöl, bezogen auf das Gesamtgewicht der Körperpeeling-Zusammensetzung;
von 5,0 bis 15,0 Gew.-% Meersalz, bezogen auf das Gesamtgewicht der Körperpeeling-Zusammensetzung; und
von 40,0 bis 56,0 Gew.-% Natriumchlorid, bezogen auf das Gesamtgewicht der Körperpeeling-Zusammensetzung,
von 0,5 bis 2,0 Gew.-% Siliziumdioxid als Suspensionsmittel, bezogen auf das Gesamtgewicht der Körperpeeling-Zusammensetzung.

2. Körperpeeling-Zusammensetzung nach Anspruch 1, wobei das Meersalz als ein Peeling in der Form von Salzkristallen mit einem durchschnittlichen Durchmesser von 0,013 cm (0,005 Zoll) bis 0,051 cm (0,02 Zoll) vorhanden ist.

3. Körperpeeling-Zusammensetzung nach einem der Ansprüche 1 bis 2, ferner umfassend:
von 1,0 bis 2,0 Gew.-% Moisturizer, bezogen auf das Gesamtgewicht der Körperpeeling-Zusammensetzung; und
von 0,1 bis 2,0 Gew.-% Konservierungsmittel, bezogen auf das Gesamtgewicht der Körperpeeling-Zusammensetzung.

4. Körperpeeling-Zusammensetzung nach Anspruch 3, umfassend von 0,1 bis 2,0 Gew.-% Vitamin E als Konservierungsmittel, bezogen auf das Gesamtgewicht der Körperpeeling-Zusammensetzung.

5. Körperpeeling-Zusammensetzung nach einem der Ansprüche 1 bis 4, umfassend einen Moisturizer, ausgewählt aus Kokosnussöl, Jojobaöl, Mangobutter, Sheabutter und Kakaobutter.

## Revendications

1. Composition de lotion exfoliante comprenant :
du chlorure de sodium comme matière première de base ;
de 5,0 à 26,0 % en poids d'huile de carthame, en fonction du poids total de la composition de lotion exfoliante ;
de 0,05 à 0,1 % en poids d'huile de marula, en fonction du poids total de la composition de lotion exfoliante ;
de 0,05 à 0,1 % en poids d'huile d'argan, en fonction du poids total de la composition de lotion exfoliante ;
de 5,0 à 15,0 % en poids de sel de mer, en fonction du poids total de la composition de lotion exfoliante ;
de 40,0 à 56,0 % en poids de chlorure de sodium, en fonction du poids total de la composition de lotion exfoliante ; et
de 0,5 à 2,0 % en poids de silice comme agent de suspension, en fonction du poids total de la composition de lotion exfoliante.

2. Composition de lotion exfoliante selon la revendication 1, dans laquelle le sel de mer comme exfoliant est présent sous la forme de cristaux de sel ayant un diamètre moyen compris entre 0,013 cm (0,005 pouce) et 0,051 cm (0,02 pouce).

3. Composition de lotion exfoliante selon l'une des revendications 1 à 2, comprenant en outre :
de 1,0 à 2,0 % en poids d'agent hydratant, en fonction du poids total de la composition de lotion exfoliante ; et
de 0,1 à 2,0 % en poids d'agent de conservation, en fonction du poids total de la composition de lotion exfoliante.

4. Composition de lotion exfoliante selon la revendication 3, comprenant de 0,1 à 2,0 % en poids de vitamine E comme agent de conservation, en fonction du poids total de la composition de lotion exfoliante.

5. Composition de lotion exfoliante selon l'une des revendications 1 à 4, comprenant un agent hydratant choisi parmi l'huile de coco, l'huile de jojoba, le beurre de mangue, le beurre de karité et le beurre de cacao.
